# EUROPEAN PATENT APPLICATION

(11) **EP 4 825 835 A1**
(43) Date of publication of application: **21.10.2026**
(21) Application number: 25824206.4
(22) Date of filing: 16.06.2025
(51) Int. Cl.: C07C 7/20, C08F 2/00, C07C 15/46, C07C 255/08, C07C 11/16, C08F 12/08, C08F 20/44, C08F 36/06

(54) **METHOD OF OPERATING REACTIVE MONOMER STORAGE TANK**

(30) Priority: 03.09.2024 KR 20240119625
(71) Applicant: LG CHEM, LTD., Yeongdeungpo-gu Seoul 07336 (KR)
(72) Inventor: CHOO, Yeon Uk, Seoul 07796 (KR); LEE, Sung Kyu, Seoul 07796 (KR); AHN, Hyun Soo, Seoul 07796 (KR)
(74) Representative: Goddar, Heinz J.
(86) International application number: PCT/KR2025/008275
(87) International publication number: WO 2026/054254

(57) **Abstract**

A method of operating a reactive monomer storage tank includes: supplying a lower discharge stream of the storage tank including a liquid reactive monomer and a polymerization inhibitor to a first heat exchanger, cooling the lower discharge stream, and then refluxing the lower discharge stream to a liquid region of the storage tank; and supplying an upper discharge stream of the storage tank including a gaseous reactive monomer to a second heat exchanger through a pressurizing device, cooling the upper discharge stream, and then refluxing the upper discharge stream to a gaseous region of the storage tank.

## Description

### [Technical Field]

### CROSS-REFERENCE TO RELATED APPLICATION(S)

This application claims priority to and the benefit of Korean Patent Application No. 10-2024-0119625 filed on September 03, 2024, the entire contents of which are incorporated herein as a part of the specification.

The present disclosure relates to a method of operating a reactive monomer storage tank, and more particularly, to an operation method capable of stably suppressing polymerization in a storage tank storing a reactive monomer even in the event of a change in an external environment.

### [Background Art]

In a reactive monomer production plant, reactive monomers are transferred, stored, managed, and shipped to a storage tank. Occasionally, polymerization within storage tanks before product shipment causes an increase in polymer content, leading to deviations from product specifications and disruptions in sales.

In addition, the polymerization of the reactive monomer may occur at a much faster rate than usual in a specific environment where there is a high temperature or a lack of polymerization inhibitor. The polymerization is an exothermic reaction, and a lot of heat is released during the polymerization process, which leads to runaway polymerization due to the heat. In addition, due to the rapid heat discharge rate, monomers or flammable substances heated above the boiling point evaporate to form explosive vapor clouds, which may lead to explosions if there is an ignition source.

A representative example of such reactive monomers may include styrene monomer (SM). The styrene monomer is a major basic oil component of petrochemicals used as a plastic synthesis raw material for polystyrene (PS), SBR, and ABS. The double bond (vinyl group) in the styrene monomer structure has the property of being easily polymerized by light, heat, peroxide, catalyst, etc., and is used as a polymer synthesis raw material. However, due to its high reactivity, the styrene monomer may cause unintended polymerization, i.e., self-polymerization, in the storage tank during storage and transportation. Periodic monitoring of a temperature of the styrene monomer, a polymerization inhibitor in the styrene monomer, and a polymer concentration in the styrene monomer to properly control the self-polymerization is very important in terms of product management.

In a plant that produces and manages reactive monomers such as the styrene monomer, the temperature inside the storage tank and the concentration of polymerization inhibitor are very important factors that are managed and operated, but the unintended temperature increase may occur due to the influence of the external environment. When the temperature change occurs due to the influence of the external temperature, etc., the polymerization may occur and the polymer may be formed, which may not only cause financial loss but may also lead to a serious accident. In addition, when the gaseous reactive monomer generated at a high temperature condenses on an inner wall of the storage tank due to the influence of the external environment such as the external temperature and stagnates in a state of no flow, a problem may occur in which a solid polymer (oxide) is generated.

Therefore, it is necessary to develop a technology that may stably suppress polymerization and prevent polymer formation even when the external environment changes during an operation of a storage tank storing a reactive monomer.

### [Disclosure]

### [Technical Problem]

The problem to be solved in the present disclosure is to provide a method of suppressing polymerization of a reactive monomer and preventing polymer formation by stably operating a storage tank storing a reactive monomer in response to changes in an external environment in order to solve the problem described in the above background art.

However, the problems to be solved by the present disclosure are not limited to the above-mentioned aspects. That is, other problems that are not mentioned may be obviously understood by those skilled in the art from the following disclosure.

### [Technical Solution]

In one general aspect, a method of operating a reactive monomer storage tank includes: supplying a lower discharge stream of the storage tank including a liquid reactive monomer and a polymerization inhibitor to a first heat exchanger, cooling the lower discharge stream, and then refluxing the lower discharge stream to a liquid region of the storage tank; and supplying an upper discharge stream of the storage tank including a gaseous reactive monomer to a second heat exchanger through a pressurizing device, cooling the upper discharge stream, and then refluxing the upper discharge stream to a gaseous region of the storage tank.

### [Advantageous Effects]

According to the method of operating a reactive monomer storage tank of the present disclosure, by controlling the temperature, pressure, and/or oxygen concentration of the liquid region and the gaseous region in the storage tank in response to changes in the external environment, it is possible to stably store the reactive monomer.

More specifically, by suppressing the polymerization of the liquid reactive monomer in the storage tank to prevent the runaway polymerization due to the heat generation, and suppressing the formation of the solid polymer through the condensation of the evaporated gaseous reactive monomers on the inner wall of the storage tank, it is possible to prevent accidents such as vapor leakage and explosion.

Effects which can be achieved by the present disclosure are not limited to the above-described effects. That is, other effects that are not described may be obviously understood by those skilled in the art to which the present disclosure pertains from the following description.

### [BRIEF DESCRIPTION OF THE DRAWINGS]

FIG. 1 is a process flow diagram of a method of operating a reactive monomer storage tank according to an embodiment of the present disclosure.
FIG. 2 is a process flow diagram of the method of operating a reactive monomer storage tank according to an embodiment of the present disclosure.
FIG. 3 is a process flow diagram of a conventional method of operating a reactive monomer storage tank according to a comparative example.

### [Best Mode]

Terms and words used in the present specification and claims are not to be construed as a general or dictionary meaning but are to be construed as meaning and concepts meeting the technical ideas of the present disclosure based on a principle that the inventors can appropriately define the concepts of terms in order to describe their own inventions in best mode.

Throughout the accompanying drawings, similar or related components will be denoted by similar reference numerals.

A singular form of a noun corresponding to an item may include one or more of the item, unless the relevant context clearly dictates otherwise.

In the present disclosure, each phrase such as "A or B," "at least one of A and B," "at least one of A or B," "A, B, or C," "at least one of A, B and C," and "at least one of A, B, or C" may include any one of items listed together in the corresponding one of those phrases, or all possible combinations thereof.

A term 'and/or' includes a combination of a plurality of related described components or any one of the plurality of related described components.

Terms such as "first," "second," "1^{st}," or "2^{nd}" may simply be used to distinguish a component from another component, and do not limit the components in other respects (e.g., importance or order).

In addition, the terms 'front', 'back', 'top', 'bottom', 'side', 'left', 'right', 'upper', 'lower', etc., used in this disclosure are defined based on the drawings, and the shape and position of each component are not limited by these terms.

It will be understood that terms 'include' or 'have' specify the presence of features, numerals, steps, operations, components, parts mentioned in the present disclosure, or a combination thereof, but do not preclude the presence or addition of one or more other features, numerals, steps, operations, components, parts, or a combination thereof.

When a component is "connected," "coupled," "supported," or "in contact" with another component, this includes not only cases where the components are directly connected, coupled, supported, or in contact, but also cases where they are indirectly connected, coupled, supported, or in contact through a third component.

When a component is "on" another component, this includes not only cases where a component is in contact with another component, but also cases where there is another component between the two components.

In addition, the terms "about," "substantially," and the like used throughout the present disclosure means figures corresponding to manufacturing and material tolerances specific to the stated meaning and figures close thereto, and are used to prevent unconscionable abusers from unfairly using the disclosure of figures precisely or absolutely described to aid the understanding of the present disclosure.

In the present disclosure, the term 'stream' may mean a flow of a fluid in a process, and may also mean a fluid itself flowing in a pipe. Specifically, the stream may mean both the fluid itself and the flow of the fluid flowing within the pipe connecting each device. In addition, the fluid may include any at least one components of a gas, a liquid, and a solid.

The term "upper" used in the present disclosure means a point 0% to 20% in height downward from the top of the device, unless otherwise specified, and may specifically mean the uppermost (top). In addition, the term "lower" means a point 80% to 100% in height downward from the uppermost portion of the device, and may specifically mean the lowermost end (bottom).

In addition, "pressure" mentioned in the present disclosure means a gauge pressure measured based on atmospheric pressure.

Hereinafter, a method of operating a reactive monomer storage tank of the present disclosure will be described with reference to the attached drawings, but the attached drawings are exemplary and the scope of the method of operating the reactive monomer storage tank is not limited thereto.

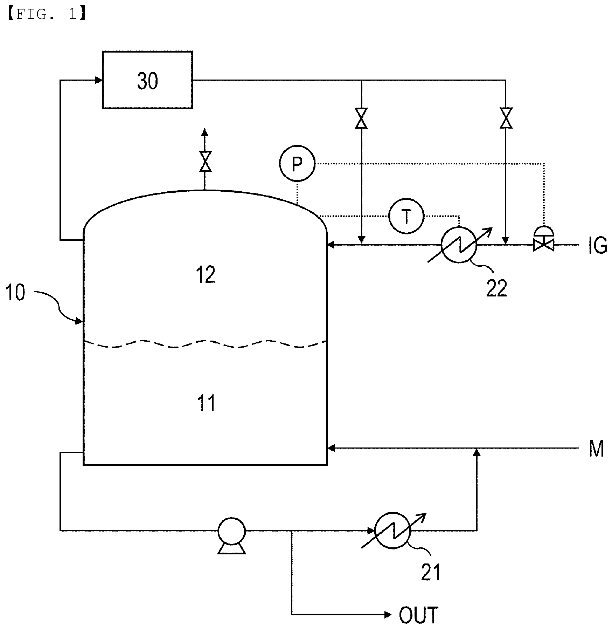

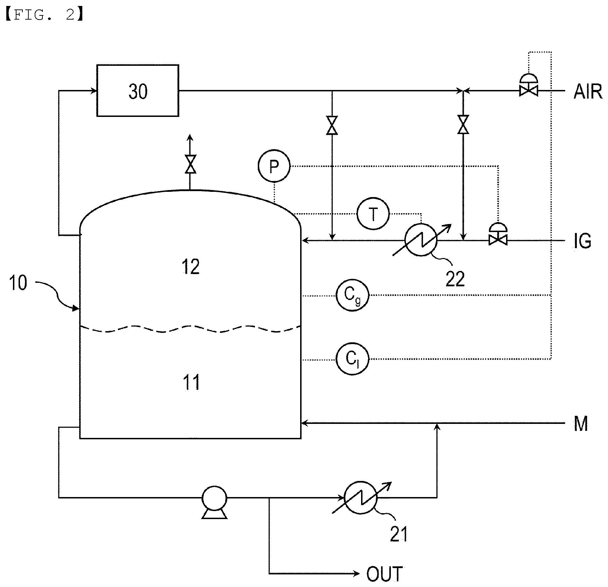

The present disclosure relates to a method of operating a storage tank storing a reactive monomer, and FIGS. 1 and 2 illustrate a process flow of operating a storage tank according to an embodiment of the present disclosure.

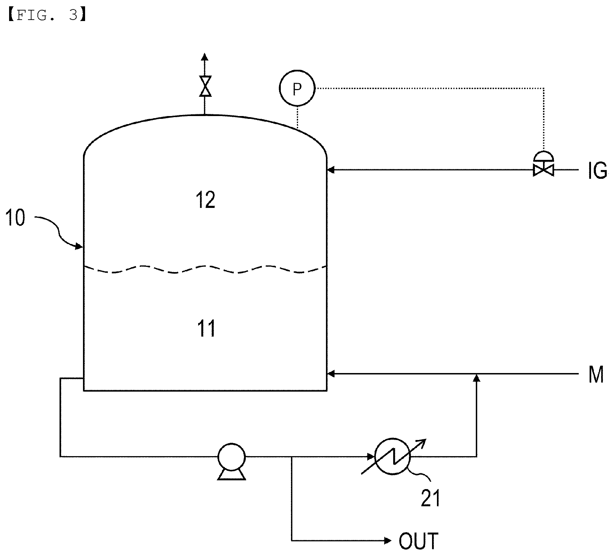

FIG. 3 illustrates a process flow of a conventional operating a conventional reactive monomer storage tank. Referring to FIG. 3, conventionally, in order to prevent an accident due to runaway polymerization when storing a reactive monomer M, it is common to use a method of supplying an inert gas IG such as nitrogen to a gaseous region 12 of a storage tank 10 to perform blanket treatment, controlling a breather valve to control the pressure inside the storage tank 10, preventing a temperature increase of a liquid reactive monomer with a heat exchanger 21, and injecting a polymerization inhibitor and managing the amount of dissolved oxygen to suppress polymer production.

Despite such management of the operating conditions of the storage tank 10, the temperature of the gaseous region 12 inside the storage tank 10 shows a tendency similar to the external temperature. Accordingly, the reactive monomer is repeatedly vaporized and condensed due to the daily temperature difference, and the gaseous region 12 without the polymerization inhibitor increases the risk of explosion as the gaseous reactive monomer increases with the temperature increase, and the reactive monomer condensed on an inner wall of the storage tank forms a dead zone where the reactive monomer stagnates without a flow, causing a problem of forming a solid polymer (oxide). Furthermore, when the polymer is formed on the inner wall of the storage tank 10 or an opening for an inlet and outlet of vapor (gas) or liquid, pressure control or temperature control becomes difficult, which may deteriorate the operational stability of the storage tank.

In order to solve such a problem, the present disclosure aims to improve the operational stability of the reactive monomer storage tank by controlling both the temperature of the liquid region and the temperature of the gaseous region in addition to the conventional operating method.

The method of operating a reactive monomer storage tank according to an embodiment of the present disclosure includes: supplying a lower discharge stream of the storage tank 10 including a liquid reactive monomer M and a polymerization inhibitor to a first heat exchanger 21, cooling the lower discharge stream, and then refluxing the lower discharge stream to a liquid region 11 of the storage tank; and supplying an upper discharge stream of the storage tank including a gaseous reactive monomer to a second heat exchanger 22 through a pressurizing device 30, cooling the upper discharge stream, and then refluxing the upper discharge stream to the gaseous region 12 of the storage tank.

Referring to FIG. 1, the lower discharge stream of the storage tank including the liquid reactive monomer M and the polymerization inhibitor is supplied to the first heat exchanger 21, cooled, and then refluxed to the liquid region 11 of the storage tank. In addition, the lower discharge stream of the storage tank 10 may be partially branched and discharged as needed, and the newly received reactive monomer M may be joined to the cooled lower discharge stream and injected into the storage tank.

The reactive monomer M is a material that is easily polymerized by light, heat, catalyst, etc., and is mainly used as a polymer raw material, and may cause self-polymerization during storage and transportation. Examples of the reactive monomer may include styrene, acrylonitrile, butadiene, etc.

In addition, most reactive monomer may exist in a liquid phase in the storage tank 10, and some may exist in a gaseous phase. Here, the inside of the storage tank 10 may be partitioned into the liquid region 11 and the gaseous region 12 based on the liquid level of the liquid reactive monomer.

In an embodiment, the styrene monomer is a colorless or yellowish flammable liquid with an irritating odor, and is used as a monomer for the polymerization of specific polymer compounds such as polystyrene (PS) resin, ABS resin, unsaturated polyester resin, and synthetic rubber (SBR) raw materials. The styrene monomer may be polymerized using various initiators such as free radicals, cations, anions, and coordination mechanisms, and the polymerization may occur by heat if there are no impurities. In addition, when the polymerization inhibitor is an appropriate ratio or less, the styrene monomer may be self-polymerized, and the temperature and pressure of the corresponding equipment may increase due to the heat generated by the polymerization. In addition, the styrene monomer reacts with oxidizers, peroxides, strong acids, etc., and copper and copper alloys should be avoided, and rust may promote polymerization. In addition, the styrene monomer has the characteristics of emitting acrid vapor during thermal polymerization and generating carbon monoxide during incomplete combustion.

The polymerization inhibitor may prevent polymerization by reacting with the free radicals of the growing chain initiated by the reactive monomer to inactivate the free radicals of the growing chain or by acting as an antioxidant that reacts with oxides generated within the reactive monomer to inhibit polymerization. For example, the polymerization inhibitor may include 4-tert-butylcatechol (TBC), p-tert-butylcatechol, etc. As an example, the TBC may be added to prevent the polymer generation due to oxidative degradation of styrene and polymerization during transportation and storage.

In addition, based on the total amount of reactive monomer contained in the liquid region of the storage tank, the content of the polymerization inhibitor may be 0.0005 wt% to 0.5 wt%, preferably 0.001 wt% to 0.002 wt%, and more preferably 0.001 wt% to 0.0015 wt%. By satisfying the content range, the polymerization of the reactive monomer may be reduced.

The storage tank 10 is a large-capacity storage facility for reactive monomers, and it is preferable that the internal upper structure is small and made of a smooth material in order to minimize the space where the polymer may be formed. For example, it is preferable to use a storage tank with minimized internal beams, pipes, and crevices that may provide a space where the vapor of the condensed reactive monomer may accumulate and polymerize. In addition, the storage tank should be made of a material that does not react with the reactive monomer or the polymerization inhibitor. For example, materials such as copper, copper alloy may react with the polymerization inhibitor to cause greenish contaminants and scale generation, which may act as a catalyst for the reactive monomer.

According to an embodiment, the liquid lower discharge stream of the storage tank is cooled by the first heat exchanger 21 and refluxed to the liquid region, thereby maintaining the temperature of the liquid region within a range of -8°C to 35°C, preferably 0°C to 24°C, and more preferably 3°C to 21°C. By maintaining the temperature of the liquid region within the above range, it is possible to suppress the polymerization of the reactive monomer.

The first heat exchanger 21 is a heat exchanger capable of liquid-liquid heat exchange, and may be, for example, a refrigerator, and its refrigerant may be freon, ammonia, propylene, cooling water, etc.

According to an embodiment, the transfer of the lower discharge stream of the storage tank 10 may be performed by a circulation pump provided in a pipe. In this case, when the lower discharge stream of the liquid phase is circulated and injected into the gaseous region 12, there is a possibility of explosion due to static electricity generation. Therefore, in order to prevent the static electricity discharge, it is preferable to provide an inlet for the liquid stream to be injected at the lower portion of the storage tank so that the lower discharge stream of the liquid phase is refluxed only to the liquid region 11.

In addition, an eductor (not illustrated) may be provided at the inlet through which the lower discharge stream of the liquid phase is refluxed. When the eductor is provided, it may help with mixing in the liquid region 11 of the storage tank, thereby improving the uniformity of temperature. Furthermore, when the discharge port and the inlet of the lower discharge stream of the liquid phase are provided in opposite directions, the mixing of the liquid region 11 may be promoted.

Referring to FIG. 1, the upper discharge stream of the storage tank including the reactive monomer in the gas phase is supplied to the second heat exchanger 22 through the pressurizing device 30, cooled, and then refluxed to the gaseous region 12 of the storage tank. In this case, the pressure and temperature of the gaseous region 11 of the storage tank 10 may be monitored in real time by a pressure measuring sensor P and a temperature measuring sensor T, respectively, and the refrigerant of the second heat exchanger 22 may be a liquid such as water.

If necessary, the upper discharge stream of the storage tank may be branched, and some of the upper discharge stream may be refluxed to the storage tank 10 without passing through the second heat exchanger 22, and the remainder may be cooled through the second heat exchanger 22 and then refluxed to the storage tank 10.

According to an embodiment, the upper discharge stream of the gaseous phase of the storage tank may be cooled by the second heat exchanger 22 and refluxed to the gaseous region 12, thereby maintaining the temperature of the gaseous region within a range of 0°C to 35°C, preferably 0°C to 30°C, and more preferably 0°C to 25°C. By maintaining the temperature of the gaseous region within the above range, the evaporation of the liquid reactive monomer within the storage tank may be prevented, and thus, the formation of a solid polymer (oxide) by the reactive monomer condensed on the inner wall of the storage tank due to influences such as the external temperature may be suppressed.

In addition, the upper discharge stream of the gaseous phase of the storage tank 10 may be transported by the pressurizing device 30. In this case, when the upper discharge stream of the gaseous phase is circulated and injected into the liquid region 11, the vapor may move from the liquid region 11 to the gaseous region 12, causing droplets to splash and polymerization due to droplet entrainment. Therefore, it is preferable to reflux the upper discharge stream of the gaseous phase only to the gaseous region 12 of the storage tank.

In addition, the pressure of the gaseous region 12 may be controlled by the pressurizing device 30 and/or a breather valve provided on the upper portion of the storage tank to discharge the vapor, and for example, the pressure of the gaseous region may be maintained within a range of 0 mmH₂O to 200 mmH₂O, preferably 0 mmH₂O to 50 mmH₂O, and more preferably 10 mmH₂O to 20 mmH₂O. By maintaining the pressure of the gaseous region within the above range, the temperature of the gaseous region may be controlled and the stability of the facility may be improved. The pressurizing device 30 may be, for example, a blower or a compressor, and the breather valve may be designed to minimize the emission of vapor.

In addition, in order to prevent static electricity accidents, etc., an inert gas IG may be injected into the gaseous region 12 of the storage tank to perform blanket treatment. Specifically, referring to FIG. 1, the inert gas IG may be injected into the upper discharge stream of the storage tank and supplied to the gaseous region 12 of the storage tank. For example, the inert gas may include nitrogen (N₂), carbon dioxide (CO₂), etc., and the amount of the inert gas injected may be extremely small.

Meanwhile, in order for the polymerization inhibitor to work effectively within the reactive monomer, sufficient oxygen should be dissolved within the reactive monomer, and even when storing the reactive monomer by blanketing the reactive monomer with the inert gas, the sufficient oxygen concentration should be maintained. In this regard, referring to FIG. 2, by injecting air (AIR) into the upper discharge stream of the storage tank and refluxing the upper discharge stream to the gaseous region 12 of the storage tank, the dissolved oxygen concentration of the liquid region 11 of the storage tank and the oxygen concentration of the gaseous region 12 may be controlled. In this case, the dissolved oxygen concentration of the liquid region 11 and the oxygen concentration of the gaseous region 12 may be monitored in real time by an oxygen concentration measuring sensor (C_{g,} Cₗ). In addition, by controlling the amount of air (AIR) supplied to the storage tank 10 in real time according to the oxygen concentration of each region confirmed by the oxygen concentration measuring sensor, the dissolved oxygen concentration of the liquid region 11 and the oxygen concentration of the gaseous region 12 may be controlled. For example, when the air (AIR) is supplied to the gaseous region of the storage tank and the oxygen concentration in the gaseous region 12 increases, the dissolved oxygen concentration in the liquid region 11 may also increase.

For example, the dissolved oxygen concentration in the liquid region 11 may be controlled to 5 ppm to 30 ppm, preferably 10 ppm to 25 ppm, and more preferably 15 ppm to 20 ppm. In addition, the oxygen concentration in the gaseous region may be controlled to 2 vol% to 10 vol%, preferably 3 vol% to 8 vol%, and more preferably 5 vol% to 7 vol%. When the dissolved oxygen concentration in the liquid region 11 and the oxygen concentration in the gaseous region 12 are maintained within the above ranges, the polymerization inhibitor may effectively function to suppress the polymer production. For example, when the oxygen concentration of the gaseous region 12 is lowered below the lower limit range, the dissolved oxygen concentration of the liquid region 11 is lowered below the appropriate range, and accordingly, the action of the polymerization inhibitor on the liquid reactive monomer is stopped, so that the polymerization may proceed.

Furthermore, the air (AIR) is supplied to the gaseous region 12 of the storage tank after passing through the second heat exchanger 22 together with the gaseous upper discharge stream, thereby minimizing the temperature change of the gaseous region.

In the present disclosure, if necessary, devices such as valves, sensors, pumps, and mixers may be additionally used.

Hereinabove, the method of operating a reactive monomer storage tank according to the present disclosure has been shown in the description and drawings, but the above drawings and the description describe and illustrate only the essential components for understanding the present disclosure. In addition to the processes and device illustrated in the above description and drawings, processes and apparatus not separately described and shown may be appropriately applied and used to implement the method of operating a reactive monomer storage tank according to the present disclosure.

### [Example]

### Example 1

According to the process flow illustrated in FIG. 1, the storage tank 10 including the styrene monomer M was operated, and the 4-tert-butylcatechol (15 ppm) was used as the polymerization inhibitor.

Specifically, referring to FIG. 1, the lower discharge stream of the storage tank including the liquid reactive monomer M and the polymerization inhibitor is supplied to the first heat exchanger 21, cooled, and then refluxed to the liquid region 11 of the storage tank, thereby controlling the temperature of the liquid region. In addition, the upper discharge stream of the storage tank including the gaseous reactive monomer is supplied to the second heat exchanger 22 through the pressurizing device 30, cooled, and then refluxed to the gaseous region 12 of the storage tank, thereby controlling the temperature and pressure of the gaseous region. In addition, nitrogen was supplied to the gaseous region 12 of the storage tank to perform the blanket treatment, and the pressure of the gaseous region was additionally controlled through the breather valve on the upper portion of the storage tank.

When the storage tank was operated according to the above Example 1, the temperature of the liquid region 11 of the storage tank was maintained within the range of -8 °C to 35°C even after a long period of time, and the gaseous region 12 was able to maintain a target constant temperature within the range of 0°C to 35°C and the pressure within the range of 0 mmH₂O to 200 mmH₂O. Accordingly, it is possible to secure the operational stability of the storage tank, and suppress the polymer formation on the inner wall of the storage tank.

### Example 2

According to the process flow illustrated in FIG. 2, the storage tank 10 including the styrene monomer M was operated, and the 4-tert-butylcatechol (15 ppm) was used as the polymerization inhibitor.

Specifically, referring to FIG. 2, the storage tank was operated in the same manner as Example 1 except that the air (AIR) was supplied to the gaseous region 12 of the storage tank while the dissolved oxygen concentration of the liquid region 11 and the oxygen concentration of the gaseous region 12 were monitored in real time using the oxygen concentration measuring sensor (Cₗ, C_{g}).

When the storage tank was operated according to the above Example 2, even after a long period of time has passed, the liquid region 11 of the storage tank was able to maintain a temperature in the range of -8°C to 35°C and the dissolved oxygen concentration in the range of 5 ppm to 30 ppm, and the gaseous region 12 was able to maintain a target constant temperature in the range of 0°C to 35°C, the pressure in the range of 0 mmH₂O to 200 mmH₂O, and the oxygen concentration of 2 vol% to 10 vol%. Accordingly, it is possible to secure the operational stability of the storage tank, and suppress the polymer formation on the inner wall of the storage tank.

### Comparative Example 1

According to the process flow illustrated in FIG. 3, the storage tank 10 including the styrene monomer M was operated, and the 4-tert-butylcatechol (15 ppm) was used as the polymerization inhibitor.

Specifically, referring to FIG. 3, the storage tank was operated in the same manner as in Example 1 except that the process of cooling the upper discharge stream of the storage tank including the gaseous reactive monomer was not performed because the pressurizing device 30 and the second heat exchanger 22 were not provided.

When the storage tank was operated according to Comparative Example 1, the liquid region 11 of the storage tank maintained a temperature in the range of -8°C to 35°C, but the gaseous region 12 was greatly affected by the external temperature, making it difficult to maintain the target constant temperature. Specifically, the temperature of the gaseous region tended to be similar to the external temperature, resulting in low operational stability and difficulty in rapid temperature control.

In the foregoing, exemplary embodiments of the present disclosure have been described, but the present disclosure is not limited thereto, and those of ordinary skill in the art will be able to understand that various changes and modifications are possible within the idea and scope of the claims set forth below.

### [Description of Reference Signs]

10: Storage tank
11: Liquid region of storage tank
12: Gaseous region of storage tank
21: First heat exchanger
22: Second heat exchanger
30: Pressurizing device
P: Pressure measuring sensor
T: Temperature measuring sensor
C_{g}: Oxygen concentration measuring sensor of gaseous region
Cₗ: Dissolved oxygen measuring sensor of liquid region
M: Reactive monomer
IG: Inert gas

## Claims

1. A method of operating a reactive monomer storage tank, comprising:
supplying a lower discharge stream of the storage tank, including a liquid reactive monomer and a polymerization inhibitor, to a first heat exchanger, cooling the lower discharge stream, and then refluxing the lower discharge stream to a liquid region of the storage tank; and
supplying an upper discharge stream of the storage tank, including a gaseous reactive monomer, to a second heat exchanger through a pressurizing device, cooling the upper discharge stream, and then refluxing the upper discharge stream to a gaseous region of the storage tank.

2. The method of operating a reactive monomer storage tank in claim 1, comprising:
injecting air into the upper discharge stream of the storage tank to control a dissolved oxygen concentration in the liquid region and the gaseous region of the storage tank.

3. The method of operating a reactive monomer storage tank in claim 2,
wherein the dissolved oxygen concentration in the liquid region of the storage tank is controlled to 5 ppm to 30 ppm, and
the oxygen concentration in the gaseous region of the storage tank is controlled to 2 vol% to 10 vol%.

4. The method of operating a reactive monomer storage tank in claim 1,
wherein a temperature of the liquid region of the storage tank is maintained within a range of -8°C to 35°C, and
a temperature of the gaseous region of the storage tank is maintained within a range of 0°C to 35°C.

5. The method of operating a reactive monomer storage tank in claim 1,
wherein a pressure of the gaseous region of the storage tank is maintained within a range of 0 mmH₂O to 200 mmH₂O.

6. The method of operating a reactive monomer storage tank in claim 1, comprising:
injecting an inert gas into the upper discharge stream of the storage tank and supplying the inert gas to the gaseous region of the storage tank.

7. The method of operating a reactive monomer storage tank in claim 6,
wherein the inert gas includes at least one selected from the group consisting of nitrogen and carbon dioxide.

8. The method of operating a reactive monomer storage tank in claim 1,
wherein the reactive monomer is selected from the group consisting of styrene, acrylonitrile, and butadiene.

9. The method of operating a reactive monomer storage tank in claim 1,
wherein the polymerization inhibitor includes at least one selected from the group consisting of 4-tert-butylcatechol and p-tert-butylcatechol.

10. The method of operating a reactive monomer storage tank in claim 1,
wherein the pressurizing device is a blower or a compressor.
